# EUROPEAN PATENT APPLICATION

(11) **EP 3 517 025 A1**
(43) Date of publication of application: **31.07.2019**
(21) Application number: 18153302.7
(22) Date of filing: 24.01.2018
(51) Int. Cl.: A61B 5/00

(54) **AN APPARATUS AND ASSOCIATED METHODS FOR ADJUSTING A USER'S SLEEP**

(71) Applicant: Nokia Technologies Oy, 02610 Espoo (FI)
(72) Inventor: Jantunen,, Joni Jorma Marius, 00250 Helsinki (FI)
(74) Representative: Potter Clarkson

(57) **Abstract**

An apparatus comprising: at least one processor; and at least one memory including computer program code, the at least one memory and the computer program code configured to, with the at least one processor, cause the apparatus to perform at least the following: receive a sleep profile of a user, the sleep profile comprising recorded sleep phases of a sleep session of the user; receive a target sleep outcome for the user, and based on the sleep profile and the target sleep outcome, determine a sleep adjustment for provision to the user, the sleep adjustment comprising stimuli configured to attempt to adjust the user's sleep during one or more of the sleep session and a subsequent sleep session in an attempt to achieve, at least in part, the target sleep outcome.

## Description

### Technical Field

The present disclosure relates to apparatus and methods associated with adjusting a user's sleep habits.

Some examples may relate to portable electronic devices, in particular, so-called hand-portable electronic devices which may be hand-held in use (although they may be placed in a cradle in use). Such hand-portable electronic devices include so-called Personal Digital Assistants (PDAs) and tablet PCs. Certain portable electronic devices may be wearable, such as on the wrist. The portable electronic devices/apparatus according to one or more disclosed example aspects/embodiments may provide one or more audio/text/video communication functions (e.g. telecommunication, videocommunication, and/or text transmission, Short Message Service (SMS)/ Multimedia Message Service (MMS)/emailing functions, interactive/non-interactive viewing functions (e.g. web-browsing, navigation, TV/program viewing functions), music recording/playing functions (e.g. MP3 or other format and/or (FM/AM) radio broadcast recording/playing), downloading/sending of data functions, image capture function (e.g. using a (e.g. in-built) digital camera), and gaming functions.

### Background

Recent developments in technology include devices used to monitor a user's sleep habits.

The listing or discussion of a prior-published document or any background in this specification should not necessarily be taken as an acknowledgement that the document or background is part of the state of the art or is common general knowledge.

### Summary

According to a first aspect, there is provided an apparatus comprising:
at least one processor; and
at least one memory including computer program code,
the at least one memory and the computer program code configured to, with the at least one processor, cause the apparatus to perform at least the following:
   receive a sleep profile of a user, the sleep profile comprising recorded sleep phases of a sleep session of the user;
   receive a target sleep outcome for the user, and
   based on the sleep profile and the target sleep outcome, determine a sleep adjustment for provision to the user, the sleep adjustment comprising stimuli configured to attempt to adjust the user's sleep during one or more of the sleep session and a subsequent sleep session in an attempt to achieve, at least in part, the target sleep outcome.

The memory and computer program code may be configured to, with the processor, cause the apparatus to provide the sleep adjustment to the user.

The memory and computer program code may be configured to, with the processor, cause the apparatus to determine the sleep adjustment for provision to the user by using a sleep biosignal recorded during a sleep session of the user.

The memory and computer program code may be configured to, with the processor, cause the apparatus to determine the sleep profile of the user by using one or more of:
a sleep biosignal recorded during a sleep session of the user; and
one or more demographic factors of the user.

The sleep session during which the sleep biosignal was recorded and/or during which the sleep profile of the user is determined may be a previous sleep session or the current sleep session.

The sleep biosignal may comprise one or more of: a position of the user's head; a position of the user's body; motion of the user's head; motion of the user's body; motion of the user's eyes; the heart rate of the user; R-R intervals of the user's heartbeats; the breathing rate of the user; and the temperature of the user, during the sleep session.

The sleep biosignals may be captured using one or more of: a device located with the user, a microphone (e.g. to detect the user's breathing and/or snoring, and/or to detect ambient sounds), an accelerometer, a gyroscope, a posture detector, a motion detector, a thermometer (to measure the temperature of the user's body, the user's skin, and/or the ambient temperature), a heart rate monitor (optical and/or electrical), an electrocardiogram (ECG), a photoplethysmography device (PPG) such as a pulse oximeter, a ballistocardiography device (BCG), a skin conductivity detector, a skin moisture detector, a body impedance detector, a device located remote from the user, a sleep monitor, a camera, an infra-red (IR) sensor, and radar.

The apparatus may comprise one or more of: an accelerometer, a gyroscope, a thermometer, a microphone, a posture detector, a motion detector, a skin conductivity detector, a skin moisture detector, a body impedance detector and a heart rate monitor, configured to determine one or more sleep biosignals.

The target sleep outcome may be one or more of:
determined by the apparatus based on the received sleep profile;
input by the user; and
input by a secondary user other than the user.

The target sleep outcome may be one or more of:
sleeping for a different total sleep time, in a subsequent sleep session, than the sleep profile total sleep time;
sleeping for the same total sleep time, but for a different portion of the day, in a subsequent sleep session, compared with the portion of the day spent sleeping of the sleep profile;
changing the length of time of one or more sleep phases;
changing the length of time of one or more sleep phases in a subsequent sleep session compared with the corresponding sleep phases of the sleep profile; and
reducing the duration of one or more detected adverse sleep conditions (e.g. snoring, sleep apnoea).

In examples where a subsequent sleep session is to be attempted to be adjusted according to the target sleep outcome, the sleep profile comprises at least one previous sleep session.

The memory and computer program code may be configured to, with the processor, cause the apparatus to receive awake profile data for the user, the awake profile data comprising a record of activities during a period of being awake; and determine the sleep adjustment further based on the awake profile data for the user.

The sleep adjustment may comprise stimuli configured to attempt to adjust the user's sleep during one or more of the sleep session and the subsequent sleep session by attempting to induce an adjustment in sleeping posture of the user.

The stimuli may be configured to be provided to the user by one or a pair of earphones to be worn by the user during one or more of the sleep session and the subsequent sleep session.

The apparatus may be one of: an earphone, a module for an earphone, a server remote from and in communication with an earphone; the earphone to be worn by the user during one or more of the sleep session and the subsequent sleep session; or a pillow.

The sleep adjustment may comprise one or more of: audio stimuli, vibratory stimuli, temperature stimuli, and pressure stimuli.

The memory and computer program code may be configured to, with the processor, cause the apparatus to determine an awake instruction for provision to the user, the awake instruction indicating an activity for the user to perform to support the attempt to adjust the user's sleep during one or more of the sleep session and the subsequent sleep session in the attempt to achieve, at least in part, the target sleep outcome.

The audio stimuli may comprise one or more of: music, tones, speech, and sounds from nature.

The memory and computer program code may be configured to, with the processor, cause the apparatus to determine the identity of the particular user by determining one or more biosignals indicative of the particular user, and determine a sleep adjustment for the particular user.

The memory and computer program code may be configured to, with the processor, cause the apparatus to determine whether the duration of provision of the sleep adjustment exceeds a predetermined sleep adjustment threshold, and if it does, prevent the provision of further sleep adjustment until the expiry of a sleep adjustment cooling off period. The predetermined sleep adjustment threshold may be a predetermined number of consecutive sleep sessions.

In a further aspect there is provided a computer-implemented method comprising:
receiving a sleep profile of a user, the sleep profile comprising recorded sleep phases of a sleep session of the user;
receiving a target sleep outcome for the user, and
based on the sleep profile and the target sleep outcome, determining a sleep adjustment for provision to the user, the sleep adjustment comprising stimuli configured to attempt to adjust the user's sleep during one or more of the sleep session and a subsequent sleep session in an attempt to achieve, at least in part, the target sleep outcome.

The steps of any method disclosed herein do not have to be performed in the exact order disclosed, unless explicitly stated or understood by the skilled person.

Corresponding computer programs for implementing one or more steps of the methods disclosed herein are also within the present disclosure and are encompassed by one or more of the described examples.

In a further aspect there is provided a computer readable medium comprising computer program code stored thereon, the computer readable medium and computer program code being configured to, when run on at least one processor, perform:
receiving a sleep profile of a user, the sleep profile comprising recorded sleep phases of a sleep session of the user;
receiving a target sleep outcome for the user, and
based on the sleep profile and the target sleep outcome, determining a sleep adjustment for provision to the user, the sleep adjustment comprising stimuli configured to attempt to adjust the user's sleep during one or more of the sleep session and a subsequent sleep session in an attempt to achieve, at least in part, the target sleep outcome.

One or more of the computer programs may, when run on a computer, cause the computer to configure any apparatus, including a circuit, controller, or device disclosed herein, or perform any method disclosed herein. One or more of the computer programs may be software implementations, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

One or more of the computer programs may be provided on a computer readable medium, which may be a physical computer readable medium such as a disc or a memory device, may be a non-transient medium, or may be embodied as a transient signal. Such a transient signal may be a network download, including an internet download.

In a further aspect there is provided a system comprising:
a control apparatus; and
one or a pair of earphones in communication with the control apparatus;
the control apparatus configured to:
receive a sleep profile of a user, the sleep profile comprising recorded sleep phases of a sleep session of the user;
receive a target sleep outcome for the user,
based on the sleep profile and the target sleep outcome determine a sleep adjustment for provision to the user, the sleep adjustment comprising stimuli configured to attempt to adjust the user's sleep during one or more of the sleep session and a subsequent sleep session in an attempt to achieve, at least in part, the target sleep outcome; and
provide the determined sleep adjustment to the one or a pair of earphones;
the one or a pair of earphones configured to:
receive the determined sleep adjustment comprising the stimuli from the control apparatus, and
provide the stimuli to the user during the one or more of the sleep session and the subsequent sleep session.

In a further aspect there is provided an apparatus comprising means for:
receiving a sleep profile of a user, the sleep profile comprising recorded sleep phases of a sleep session of the user;
receiving a target sleep outcome for the user, and
based on the sleep profile and the target sleep outcome, determining a sleep adjustment for provision to the user, the sleep adjustment comprising stimuli configured to attempt to adjust the user's sleep during one or more of the sleep session and a subsequent sleep session in an attempt to achieve, at least in part, the target sleep outcome.

The present disclosure includes one or more corresponding aspects, examples or features in isolation or in various combinations whether or not specifically stated (including claimed) in that combination or in isolation. Corresponding means for performing one or more of the discussed functions are also within the present disclosure.

The above summary is intended to be merely exemplary and non-limiting.

### Brief Description of the Figures

A description is now given, by way of example only, with reference to the accompanying drawings, in which:
Figure 1 shows a schematic of a sleep cycle;
Figure 2 shows an example apparatus according to the present disclosure;
Figure 3 shows different sleeping postures;
Figure 4 shows an example audio output device according to the present disclosure;
Figure 5 shows an example system according to the present disclosure;
Figure 6 shows an example apparatus according to the present disclosure;
Figure 7 shows an example of sleep phases experienced by a user during a sleep session;
Figure 8 shows an example system according to the present disclosure;
Figure 9 shows an example system according to the present disclosure;
Figure 10 shows an example method for determining sleep adjustments for a user according to the present disclosure;
Figure 11 shows an example method according to the present disclosure; and
Figure 12 shows a computer-readable medium comprising a computer program configured to perform, control or enable the method of Figure 11.

### Description of Specific Examples

Recent developments in technology include devices used to monitor a user's sleep habits.

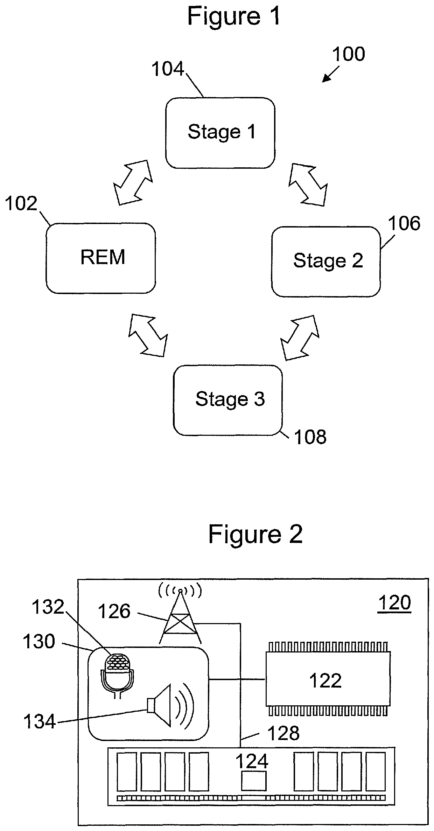

Figure 1 shows a schematic of a sleep cycle 100. From being awake, first a user going to sleep may enter REM (rapid eye movement) sleep 102. REM sleep revitalises the memory. In this stage, brain activity is very high, and intense. Dreaming is likely to occur. REM sleep occurs first in about the first 90 minutes after falling asleep. From REM sleep the user may enter Stage 1 sleep 104. In Stage 1 sleep a user can experience a light transitional sleep. This stage is where drowsiness and sleep begin. In some cases, a user may enter Stage 1 sleep followed by REM sleep. From Stage 1 sleep the user can enter Stage 2 sleep 106. In Stage 2 sleep, more stable sleep occurs. Chemicals produced in the brain block the senses, making it difficult to be woken. From Stage 2 sleep a user can enter Stage 3 sleep 108, which is deep sleep. Growth hormone is released during this stage. Most Stage 3 sleep occurs in the first third of the night. A user may be woken back through Stages 2, 1 and REM sleep from Stage 3, or may skip one or more stages to be woken. Waking directly from certain stages (such as Stage 2 106 and Stage 3 108 sleep) may leave the user feeling unrefreshed and drowsy. A user being woken from REM 102 or Stage 1 104 sleep may feel more refreshed because they are naturally in a lighter sleep state before being awoken. A user may also feel more refreshed following a sleep session if they have experienced sufficient proportions of the different stages of sleep. Further, experiencing good quality sleep, including sufficient time in certain stages of sleep, may be associated with improved cognitive function and memory following the sleep session. The terms "sleep stage" and "sleep phase" are synonymous.

Examples disclosed herein may aid a user to have improved sleep, and may allow the user to attempt to adjust their sleep habits and patterns in an attempt to achieve, at least in part, a particular target sleep outcome, such as feeling more awake upon waking, achieving a set minimum amount of time of deep sleep, or improving cognitive function, for example.

Figure 2 shows an apparatus 120 as disclosed herein. The apparatus 120 may, in some examples, be an earphone, or a module for an earphone. The apparatus 120 may, in some examples, be a server remote from and in communication with an earphone, wherein the earphone is to be worn by a user during a sleep session. In some examples the apparatus 120 may be a pillow (i.e. a head/neck support to be used whilst sleeping).

The apparatus 120 comprises a processor 122 and memory 124 (including computer program code) and a transceiver 126, which are electrically connected to one another by a data bus 128. The apparatus 120 of Figure 2 also comprises an audio interface 130 comprising at least one microphone 132 configured to receive audio input, and at least one audio speaker 134 (here as part of an earphone) configured to provide audio output. In some examples, the apparatus 120 may not comprise the audio interface 130, or may comprise the speaker 134 of the audio interface 130 but not the microphone 132. In some examples the audio device 134 may be an earphone/pair of earphones, or may be a speaker configured, for example, to sit on a bedside table or under a pillow. In examples where stimuli other than, or as well as, audio stimuli may be provided, the apparatus 120 may comprise one or more of a temperature changing element (e.g. a warmable/coolable element), a pressure changing element (e.g. an inflatable element), and a vibratory element (e.g. a vibrating wearable element). The apparatus in some examples may be in communication with one or more of an audio output device, a temperature changing element (e.g. an electric heating blanket), a pressure changing element (e.g. an inflatable mattress or pillow) and a vibratory element (e.g. a vibratable mattress cover).

The at least one memory 124 and the computer program code are configured to, with the at least one processor 122, cause the apparatus 120 to receive a sleep profile of a user, the sleep profile comprising recorded sleep phases of a sleep session of the user; receive a target sleep outcome for the user, and based on the sleep profile and the target sleep outcome, determine a sleep adjustment for provision to the user. For example, the user's sleeping habits (the duration and timing of the different phases of sleep) may be recorded (by the apparatus or by one or more separate apparatuses). The user may have a particular target which they wish to achieve by tuning their sleep habits (e.g. they wish to awake at 6am feeling refreshed, or they wish to achieve at least eight hours "good quality" sleep a night (i.e. a healthy balance of sleep phases at appropriate times)). The apparatus can determine a "sleep adjustment" to provide to the user to help achieve the target sleep outcome.

The sleep adjustment in this example comprises audio stimuli configured to attempt to adjust the user's sleep during one or more of the sleep session and a subsequent sleep session in an attempt to achieve, at least in part, the target sleep outcome. In some examples, the apparatus 120 may be configured to provide the sleep adjustment to the user, for example, as an audio stimulus provided via the speaker(s) 134 of the audio interface 130. It is recognised that the stimuli provided to the user may not always cause the user's sleep during a sleep session to be adjusted in the expected manner. Therefore, achieving the target sleep outcome may not always be fully realised for the user. However, by providing the determined sleep adjustment as described herein, a reasonable attempt is made to adjust the user's sleep during a sleep session in order to help attempt achieve, at least in part, the target sleep outcome. For example, a target sleep outcome of "sleep one hour longer" may not be fully realised for the user, if the user wakes up before the extra one-hour elapses. However, the provided sleep adjustment stimuli may have still helped the user to sleep longer (e.g. 50 minutes), thus achieving, at least in part, the target sleep outcome.

The processor 122 may be configured for general operation of the apparatus 120 by providing signalling to, and receiving signalling from, the other components to manage their operation. The memory 124 may be configured to store computer code configured to perform, control or enable operation of the apparatus 120. The memory 124 may also be configured to store settings for the other components (including, for example, a microphone 132 configured to receive audio signals from the user, and/or the speaker 134 configured to provide audio signals/stimuli to the user). The processor 122 may access the memory 124 to retrieve the component settings in order to manage the operation of the other components.

The transmitter 126 may comprise a separate transmitter and receiver and be configured to transmit data to, and receive data from, one or more other devices via a wireless or a wired connection. For example, if the apparatus 120 forms part of a system, the transceiver 126 may be configured to receive information from a remote server or analysis module providing information on stimuli to provide to the user during sleep, and/or transmit information to a remote server or analysis module providing information on audio and/or sensor signals received from the user during sleep, so that the remote server or analysis module may determine the user's sleep phases during a sleep session.

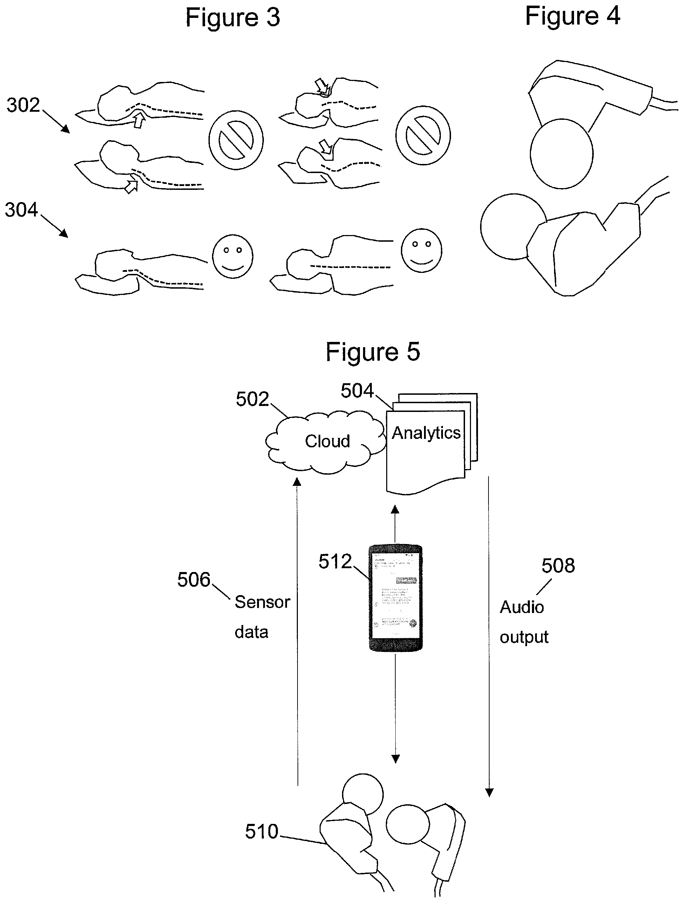

Figure 3 shows different sleeping postures. Certain postures 302 may be detrimental to achieving a good quality relaxing period of sleep, for example because pressure is placed on the user's neck or shoulders which causes the user to awake feeling tense or unrelaxed. Certain postures 304 may be conducive to achieving a good quality relaxing period of sleep, for example because the user's head, neck and shoulders are positioned and supported in a natural balanced way, allowing the user to sleep and fully relax their upper body muscles, to awake feeling rested and refreshed.

Audio stimuli to be provided to the user to achieve the user's target sleep outcome may cause the user, during sleep, to adjust their body posture and rest in a more relaxing posture 304 to help achieve improved quality sleep. For example, providing an unpleasant audio stimulus in the user's right ear may cause the user to roll over from their right side (in an uncomfortable posture 302) to their back (in a comfortable posture 304), allowing the user to more easily enter a deeper sleep, at which point the audio stimulus may cease, allowing the user to sleep in a quiet environment. Apparatus disclosed herein such as the apparatus described in relation to Figure 2 may be configured to determine (and, in some examples, provide) a sleep adjustment comprising stimuli, such as audio stimuli, which are configured to attempt to adjust the user's sleep during the sleep session by inducing an adjustment in sleeping posture of the user. The sleep session may be a current sleep session, e.g. in which the user's poor sleep posture is detected and attempted to be improved just after the time of detection in the same sleep session, through the provision of stimuli. The sleep session may be a subsequent sleep session, for example, following detection for three nights of the user snoring, it may be determined that the snoring is a persistent habit and in the next sleep session, the apparatus attempts to reduce the snoring, for example, by inducing a change in sleeping posture of the user through the provision of stimuli. Such apparatus may also comprise, or be in communication with, one or more sensors configured to detect the current posture of the apparatus and provide this information as feedback, so the apparatus is provided with the user's posture as input. Thus, the apparatus receives e.g. input indicating the position of the user, so if the audio stimuli is intended to induce a posture change, the apparatus can be informed of how and when the posture change has taken place.

Figure 4 shows an example audio output device according to the present disclosure. In some examples, audio stimuli may be provided to the user by one or a pair of earphones to be worn by the user during a sleep session. In other examples, audio stimuli may be provided by a standalone (non-earphone) speaker or group of speakers. A user who shares a room or bed with another person may prefer using in-ear earphones to avoid disturbing the sleep of the other person. A person who sleeps alone may prefer receiving audio stimuli via a non-earphone based speaker, so they do not have to wear any apparatus while sleeping. Earphones may be wired, half-wired (in which the two earphones are connected to each other by a wire but may be in wireless communication with a control unit), or may be wireless (wireless or half-wired earphones may be preferred to allow for movement during sleep without the possibility of pulling/tangling the earphone wires). Wireless earphones may require two separate transceivers, one for the left earphone and another for the right earphone. In-ear earphones such as those shown in Figure 4 may be "noise cancelling" and thus configured to decrease the volume of ambient noise detected by the user's ears. In some examples, the audio stimuli may be provided by way of active noise cancellation, in an attempt to attenuate any background noise and allow the user to sleep in a quieter environment.

Figure 5 shows an example system according to the present disclosure. This example shows a system comprising an audio/sensor device (earphones) 510 configured to provide the user with audio stimuli during sleep. An analysis module 502, 504, which may be a remote server 504, or may be the "Cloud" 502, is in communication 508 with the audio output device, indicating audio stimuli to provide to the user. Also in this example, sensor data 506 (e.g. audio data detected using a microphone, gyroscope data indicating a sleeping posture) from the sleeping user is provided from the audio/sensor device 510 to the analysis module 502, 504.

Figure 5 also indicates an intermediary apparatus 512, which may, for example, be a user device such as a smartphone or tablet computer. This intermediary device 512 may be configured to receive data (e.g. posture and sleep phase data) from the audio/sensor device 510 for analysis and/or provision to the analysis module 502, 504, and/or configured to receive data (e.g. an indication of audio stimuli to provide to the user) from the analysis module 502, 504 for provision to the audio/sensor device 510.

In some examples, the target sleep outcome may be determined by the apparatus based on the received sleep profile. For example, the audio/sensor device 510 may detect the user's phases of sleep during one or more previous sleep sessions, and from these determine a user's sleep pattern. The apparatus may be configured to determine the sleep profile for the user by using one or more sleep biosignals recorded during one or more previous sleep sessions and/or a current sleep session of the user. A sleep biosignal may comprise one or more of: a position of the user's head; a position of the user's body; motion of the user's head; motion of the user's body; motion of the user's eyes; the heart rate of the user; R-R intervals of the user's heartbeats and variations thereof; the breathing rate of the user; the skin conductivity of the user, and the temperature of the user during the sleep session.

For example, a user's sleep profile may show that they regularly take a long time (e.g. longer than the average time for a user of a matching demographic) to achieve Stage 2 or Stage 3 sleep (a deep sleep). The time taken to fall asleep may be detected, for example, by one or more sensors (for example by a microphone) measuring background noise levels. Background noise may decrease, or may change character, from when the user is awake (e.g. breathing more quickly and/or loudly, and moving around in bed) and asleep (quieter and/or slower breathing, and less body movement). Then, the target sleep outcome may be to achieve Stage 2 or Stage 3 sleep in a shorter time following the start of a sleep session, as this may help the user achieve a more refreshing overall sleep session. As another example, if a user is detected to regularly wake up between the hours of 3am and 7am when the user would wish to be asleep (since they have an alarm set to awake fully for 7am) then the target sleep outcome may be to help the user maintain some level of sleep deeper than REM sleep (Stages 1-3 of sleep) after 3am. Maintaining sleep may be attempted by the apparatus determining, and in some examples providing, audio stimuli conducive to the user being in a sleeping state during the times when they are not in a deep enough sleep.

As another example, a user may be detected to stay in REM or Stage 1 sleep for a significant (e.g. more than 50%) of the time between 6am and 7am, although the user has a wake-up alarm set for 6am. This may indicate that the user struggles to wake up and get out of bed when required. The target sleep outcome may therefore be to ensure the user is fully awake and can get out of bed at 6am. This may be attempted by the apparatus determining, and in some examples providing, audio stimuli to wake the user up more fully at 6am, or by suggesting the user goes to bed earlier than normal and providing audio stimuli to induce sleep at the earlier time.

As a further example, a user may be determined to snore during Stage 1 sleep and then move to lighter REM sleep, when the user may benefit from stopping snoring to help them move from Stage 1 to a deeper Stage 2 sleep. The apparatus may determine (and may provide) audio stimuli to counteract the adverse health condition of snoring and induce the user to e.g. change posture to stop snoring, and move into a deeper sleep. Other possible health-related adverse sleep conditions include sleep apnoea, insomnia, sleep deprivation, nightmares, night terrors, and restless legs syndrome (Willis-Ekbom disease).

In some examples, the apparatus may mine data from one or more other user applications to suggest and/or set a target sleep outcome for the user. For example, the apparatus may have access to the user's calendar and determine if the user has an upcoming early morning or late night meeting, or is scheduled to move to a different time zone, and suggest a target sleep outcome to help the user feel better for the scheduled event. As another example, a business traveller may wish to schedule sleep time/naps using the apparatuses discussed herein, to attempt to sleep during a flight but be awake during taxi travel and while in the airport. As another example, the apparatus may obtain a log of personal device usage of the user, and if the user is, for example, using their device during an expected sleep session, this may be an indication that the user may benefit from audio stimuli to help the user maintain sleep during the sleep session rather than being awake and using the electronic device.

In some examples, the target sleep outcome may be determined by input by the user. For example, the user may provide input instructing the apparatus to determine audio stimuli to help the user fall asleep quicker (e.g. if the user sometimes struggles to get to sleep), wake up at a certain time pre-set by the user, prolong one or more sleep phases, or determine audio stimuli to cause the user to feel more refreshed, improve their memory, have a deeper sleep, sleep in a more comfortable position (e.g. if the user sometimes wakes up with a stiff neck). The apparatus may determine audio stimuli to provide a user with a set target sleep outcome based on one or more previous sleep sessions of the user. For example, if the apparatus receives input indicating that the user generally goes to bed at 10pm but does not fall into a sleep deeper than Stage 1 sleep until midnight, the apparatus may determine that relaxing audio stimuli should be provided between 10pm and 11pm to induce a deeper sleep more quickly in the user. This may help the user achieve the target sleep outcome of "feel more refreshed when I awake". As another example, a user may wish to try and learn during their sleep, and so their target sleep outcome may be to improve their knowledge or ability in a particular subject e.g. speaking a foreign language or another subject such as history. Such a user may, for example, then receive audio stimuli comprising learning/revision material (e.g. foreign language tuition/conversation, a spoken recitation of historical events) during the phases of sleep most conducive to the user retaining the audio information received during sleep.

In some examples, the target sleep outcome may be determined by input by a secondary user other than the user. For example, a doctor may wish to help improve a patient's (user's) sleep, a relative or carer may wish to improve a user's sleep (e.g. a person may wish to help an elderly relative get better quality sleep), or a sports coach/manager may wish to help a sportsperson achieve a particular sleep goal (e.g. be awake and refreshed for a match/competition the next day). A secondary user may wish to provide a target sleep outcome for a plurality of users. For example, a sports coach may wish each member of the football team that they coach to be awake and refreshed the next day at 7am to travel to and take part in a football match.

Some user-set and secondary user-set target sleep outcomes may be pre-set in the apparatus/device (e.g. "I want to feel more refreshed", "I want to be more alert when I wake up", "I want to prepare for a change in time zone of ± X hours in Y days' time"). Other user-set and secondary user-set target sleep outcomes may be freely entered (not pre-set), such as "please wake me up at X o'clock tomorrow", "I am flying to China in three days' time, please attempt to adjust my sleep pattern so I don't feel so jet-lagged", or "my wife is tired, please help me get to sleep early tonight so I can get up early to help at home".

As mentioned above, there are many different scenarios in which a user may benefit from receiving audio stimuli to attempt to achieve a particular target sleep outcome. In general, the target sleep outcome may be one or more of: sleeping for a different total sleep time, in a subsequent sleep session, than the sleep profile total sleep time (e.g. the user usually sleeps for six hours and an attempt is made to have the user sleep for seven hours); sleeping for the same total sleep time, but for a different portion of the day, in a subsequent sleep session, compared with the portion of the day spent sleeping in the sleep profile (e.g. the user usually wakes at 6am but wishes to adjust to a time zone one hour behind, so an attempt is made to have the user become used to waking at 5am); changing the length of time of one or more sleep phases (e.g. an attempt is made to increase the length of time spend in Stage 2 and Stage 3 (deep) sleep); changing the length of time of one or more sleep phases in a subsequent sleep session compared with the corresponding sleep phases of the sleep profile (e.g. the user usually spends 30 minutes in Stage 1 sleep so an attempt is made to reduce that time to 15 minutes spent in Stage 1 sleep); and reducing the duration of one or more detected adverse sleep conditions (e.g. attempting to minimise snoring or sleep apnoea).

In some examples, the apparatus may determine the sleep adjustment further based on a determined awake profile for the user. The awake profile indicates representative activities undergone by the user during a previous period of being awake. For example, if a user regularly eats a large evening meal late at night (e.g. 10pm) the apparatus may determine that the user would benefit from going to bed at least an hour after eating, e.g. at 11.30pm. As another example, if a user takes part in a sports match during the daytime, the user may benefit from going to bed an hour earlier than usual and sleeping for an hour longer than usual to help recover from the physical exertion of the sports match.

In some examples, additionally or alternatively to the audio stimuli provided to help achieve a target sleep outcome, the apparatus may determine that the sleep adjustment comprises one or more of: vibratory stimuli, temperature stimuli, and pressure stimuli. Such stimuli are configured to attempt to adjust the user's sleep during the sleep session in an attempt to achieve the target sleep outcome, at least in part. For example, the user may use a pillow which can be controlled to change temperature, and the apparatus may provide a signal to the pillow to change temperature to help the user achieve a target sleep outcome (e.g. cool the pillow down on a hot night to help the user achieve the target sleep outcome of getting into a deeper sleep more quickly). As another example, the apparatus may provide a signal to a mattress which can vibrate in particular regions to help the user change posture and achieve a target sleep outcome (e.g. a target of snoring less, by being induced, by audio stimuli provided to the user's right ear and vibrations from the mattress under the right side of the user's body, to roll over onto the left side of the body). As another example, an in-ear earphone may be configured to inflate and deflate, and the pressure changes provided to the user's ear by the in-ear earphone may be used alongside audio stimuli to help the user e.g. change posture, or help block out detected ambient noise, to attempt to achieve a target sleep outcome. As another example, a pillow configured to change shape, such as a pillow comprising a matrix of inflatable/deflatable cells, may be used to control the pressure experienced at different regions of the user's head and neck during sleep (e.g. in a similar way to a pressure controllable mattress used to treat patients suffering from bedsores). The pressure changes provided to the user's head and neck by the pillow may help the user change posture to attempt to achieve a target sleep outcome. The pillow shape may be controlled based on one or more of the current detected posture and pressure of the user during sleep, and/or previous head and neck postures determined and recorded in the user's sleep profile. For convenience, many of the present examples refer primarily to the provision of the audio stimuli. However, it is to be appreciated, that one or more of the temperature stimuli, vibratory stimuli, and pressure stimuli may be provided additionally or alternatively to the audio stimuli.

In some examples, the apparatus may be in communication with a home automation system (or an "Internet of Things" system) in which household appliances and devices can communicate with each other. The apparatus may, for example, provide audio stimuli to help a user wake up, and in combination with these stimuli, the apparatus may send a signal to a connected air conditioning system, or heating system (for example) to change the air temperature of the room so that it is at an appropriate temperature for the user to wake up in a pleasant environmental temperature. As another example, the apparatus may provide a signal to a connected radio or music player so that the user wakes up to playing music, to help the user wake up fully and not drift back off to sleep. Connected devices may be controlled by the apparatus during sleep in some examples, e.g. to change the room temperature to a temperature more conducive to remaining asleep.

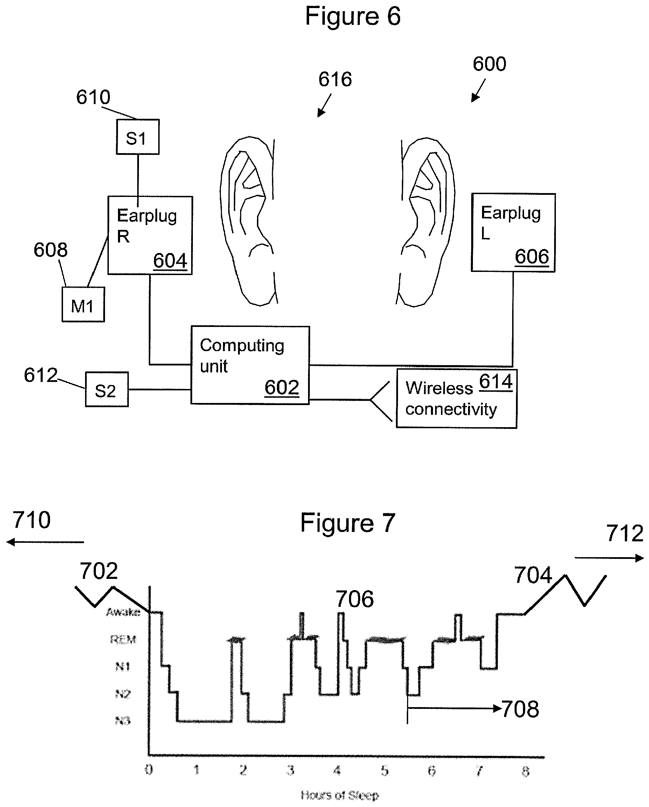

Figure 6 shows an example apparatus 600 according to the present disclosure. The apparatus 600 comprises a computing unit 602 which may be similar to the apparatus described in relation to Figure 2 (e.g. may comprise a memory and processor). The apparatus 600 comprises a right earphone 604 and a left earphonee606 to be worn by the user 616 and which are configured to output audio stimuli. In other examples there may be only one earphone 604, 606 in use. The apparatus 600 also comprises a transceiver 614 for wireless connectivity, e.g. to an external analysis unit, remote peripheral computing apparatus, or the internet. The right earphone604 is connected to a microphone sensor M1 608 which can detect the user's breathing/snoring/talking during sleep, for example, and to a sensor S1 610 which may, for example, be a temperature, motion, or posture sensor. The computing unit 602 in this example also is connected to a sensor S2 612, such as an ambient noise microphone or ambient temperature sensor. The earphones 604, 606 may generate high quality audio sounds as audio stimuli selectively to both ears (e.g. in mono, stereo, or in 3D audio). The computing unit 602 may be connected wirelessly to an application (e.g. running on a user peripheral device such as a tablet computer), and/or to the internet/Cloud. The apparatus 600 may manage data transfer (e.g. of audio output from a remote server to the earphones 604, 606), data analysis (e.g. logging a user's sleep habits), generation of audio stimuli for the user, and the selection of physical and other reactions to the provided stimuli (e.g. determining the effect of the provided audio stimuli for inclusion in a feedback process to guide the stimuli to be provided in future). The apparatus 600 is described herein as earphone(s) which may be configured to fit in the user's ear canal. However, it should be appreciated that alternative types of ear-worn apparatus 600 may be used. The alternative ear-worn apparatus 600 may be configured with the same features as described above for the earphone example. Examples of alternative ear-worn apparatus include: headphones configured to be worn on or over the user's ears; and one or a pair of in-ear earbuds configured to be worn in the user's ear but that rest outside of the user's ear canal. For convenience, we refer to the ear-worn examples of the apparatus as earphone(s) from hereon.

Figure 7 shows an example of sleep phases experienced by a user during a sleep session. Hours of sleep are plotted on the x-axis against the phase of sleep experienced (as described in relation to Figure 1). The user in this example undergoes eight hours of sleep. Different examples of target sleep outcomes are indicated in Figure 7. Prior to going to sleep 702, the user may be instructed to perform certain tasks, at certain times, to facilitate the process of getting to sleep in a way suitable for the user (e.g. taking into consideration the user's usual daily schedule). For example, a user may wish to have eight hours sleep and be awake at 6am. Thus, the user may be instructed to be in bed at 9.30pm and, for half an hour, read a book or listen to some gentle music to help the user relax. The user may then be instructed to turn off the lights at 10pm.

Following the sleep session, the user may be awoken 704 in a way which meets the user's target sleep outcome. For example, if the user wishes to awake feeling refreshed and be awake for 6am, the apparatus may provide audio stimuli to make the user up at, or just before 6am (e.g. an alarm sound gradually increasing in volume). The apparatus may, from previous sleep sessions, and/or from measuring biosignals during the current sleep session, determine that the user is undergoing REM sleep between 5.40am and 6am, and that this is a good phase of sleep to be awoken from to feel refreshed, and that the user may be awoken from these lighter sleep phases by a gentle, calm alarm sound such as birdsong. The apparatus may also similarly determine that between 6.15am and 6.45am, the user is undergoing Stage 2 or Stage 3 sleep, and that the user should not be awoken during these sleep phases because the user will likely wake up feeling unrefreshed/stressed because they were in a deep sleep, and/or may require a loud unpleasant alarm (e.g. an alarm bell or buzzer) to be awoken from these phases of sleep anyway.

During the night's sleep 706, the apparatus may provide audio stimuli to mitigate the risk of the user waking up if they are not required to. For example, the apparatus may detect (e.g. using a microphone), or receive a signal from an external apparatus indicating the detection of ambient noise (e.g. a neighbour's car alarm sounding, a group of people passing the house chatting noisily). The apparatus may determine (and in some examples, provide, e.g. via earphones) a counter-audio signal to help the user maintain their sleep. Such a signal may, for example, by a relaxing tone, nature sounds, or a background "white noise" to try and counter-balance the potential waking effect of the ambient noise. The apparatus may be configured to determine the nature of the ambient noise and, based on the determined nature, provide audio stimuli to counter-balance the ambient noise to maintain the user's sleep state (as discussed above) or provide audio stimuli to cause the user to wake gently if it is determined that the user should wake up as a result of the ambient noise (for example, if the detected noise is of a baby crying in the next bedroom, the user may receive a waking audio stimulus to cause the user to wake up and attend to the crying baby). The apparatus may aim to allow the user to sleep through noises which do not require the user's attention but be awoken for noises which may require the user's attention.

The apparatus may, in some examples, provide audio stimuli to try and prolong 708 one or more phases of the user's sleep. Figure 7 shows the apparatus has identified that it may benefit the user if their N2/Stage 2 sleep phase following 5.5 hours of sleep is prolonged. The apparatus may, for example, provide relaxing audio stimuli during this sleep phase to try and prolong it. For example, it may be that the user's target sleep outcome is to have improved focus in the mornings, and that prolonging a particular sleep phase has been indicated to help a user achieve that goal.

Preparation for a sleep session aiming for a target sleep outcome may begin prior to the user going to sleep 710. As discussed earlier, the user may be provided with instructions for activities to undertake just before going to sleep 702. In some examples the user may also receive instructions and guidance for activities to do and not do during the daytime several hours before the user is due to go to sleep. In some examples, daytime activity of the user may be provided as input to the apparatus and processed as part of the determination of audio stimuli to provide to attempt to achieve a target sleep outcome. For example, if the user has a stressful commute home between 5pm and 6pm, this high stress activity may be accounted for by the apparatus, and to help the user achieve the target sleep outcome of a refreshing night's sleep, provide particular audio stimuli (e.g. an extended "wind down" period of REM sleep and/or Stage 1 sleep) to help counteract the effect of the stressful commute home.

Similarly, the user may be provided with instructions following sleep 712 to aid the user with their target sleep outcome. For example, the user aiming to achieve the target sleep outcome of improved focus may be instructed not to use an electronic device for the first hour following waking to help the user wake gently. Also, in some examples, a target sleep outcome for the following night's sleep session 712 may be accounted for so that the audio stimuli may be provided before the following sleep session 712, for example during the day, in an attempt to help to achieve the target sleep outcome.

The sleep biosignals may be captured using one or more of: a device located with the user, a microphone (to detect the user's breathing and/or snoring, and/or to detect ambient sounds), an accelerometer, a gyroscope, a posture detector, a motion detector, a thermometer (to measure the temperature of the user's body, the user's skin, and/or the ambient temperature), a heart rate monitor (optical and/or electrical), an electroencephalogram (ECG) detector, a photoplethysmogram (PPG) detector, a ballistocardiograph (BCG) detector, a skin conductivity detector, a skin moisture detector, a body impedance detector, a device located remote from the user, a sleep monitor, a camera, an IR sensor, and radar. The apparatus may comprise one or more of these sensors/detectors.

In some examples, the apparatus may be configured to determine the identity of the particular user by determining one or more biosignals indicative of the particular user, and determine a sleep adjustment for the particular user. For example, the apparatus may be configured to provide audio stimuli via a pair of earphones, and these may be used by more than one person in the household (e.g. a husband and wife). The apparatus may be configured to determine which user is the current user, for example by the user entering a user identifier (e.g. password, passcode, fingerprint, voiceprint) and/or by the apparatus determining the identity of the user without conscious identification input from the user (e.g. by determining a skin conductivity, ear shape, fingerprint, or other bioindicator characteristic of a particular user). The identified user's profile may then be accessed (e.g. personal previous sleep profiles and/or target sleep outcomes) for determination of audio stimuli for a sleep session for that particular user. In some examples, determination of the identity of the user may be used as confirmation that the particular user has rights/permission to use the apparatus (e.g. the user has a licence/software permission).

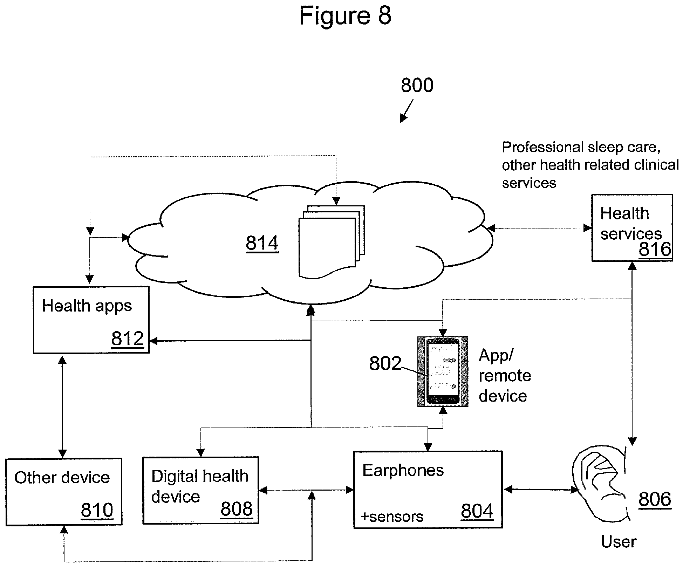

Figure 8 shows an example system 800 according to the present disclosure. The system comprises at least a control apparatus 802, and one or a pair of earphones 804 in communication with the control apparatus 802. The control apparatus 802 is configured to receive a sleep profile of a user 806, the sleep profile comprising recorded sleep phases of a sleep session of the user 806; receive a target sleep outcome for the user 806, and based on the sleep profile and the target sleep outcome, determine a sleep adjustment for provision to the user 806.The sleep adjustment comprises stimuli (in this example, audio stimuli) configured to attempt to adjust the user's sleep during one or more of the sleep session and a subsequent sleep session in an attempt to achieve, at least in part, the target sleep outcome. The control apparatus 802 is also configured to provide the determined sleep adjustment to the one or a pair of earphones 804. The one or a pair of earphones 804 is configured to receive the determined sleep adjustment comprising the stimuli from the control apparatus 802, and provide the stimuli to the user 806 during one of more of the sleep session and the subsequent sleep session.

The audio output device 804 may also comprise one or more sensors to determine signals from the user for use in determining a user's sleep habits as discussed above.

Figure 8 also shows a digital health device 808 in communication with the control apparatus 802 and the audio output device 804. A further device 810 is also shown in communication with the digital health device 808 and the audio output device 804. The digital health device(s) 808, and the further health device 810, may provide one or more additional stimuli to aid a user's sleep, such as lighting and/or may comprise one or more sensors configured to determine/monitor the user's sleep state, such as a motion sensor or ambient temperature monitor.

One or more health applications 812 may be in communication with the other device(s) 810, with the audio output device 804, and with the cloud/remote analysis module 814. Health services 816 (e.g. hospitals, doctors, clinics, therapists) may be in communication with the control apparatus 802, with the cloud/analysis module 814, and with the user 806, to determine a user's sleep habits/ issues and provide advice to the user 806. Such services 816 may be professional sleep care services, and other health care related services. Thus, data regarding the user such as the user's sleep profile, target sleep outcome, and/or one or more detected biosignals from the user may be provided securely to the health services 816 to aid in the provision of healthcare and lifestyle management of the user.

The cloud/analysis module 814 may determine the user's sleep phases during a sleep session from data recorded by one or more sensors e.g. with the audio output device 804, and/or may determine and/or provide audio stimuli (and any other additional stimuli) to provide to the user during a sleep session to attempt to achieve the target sleep outcome.

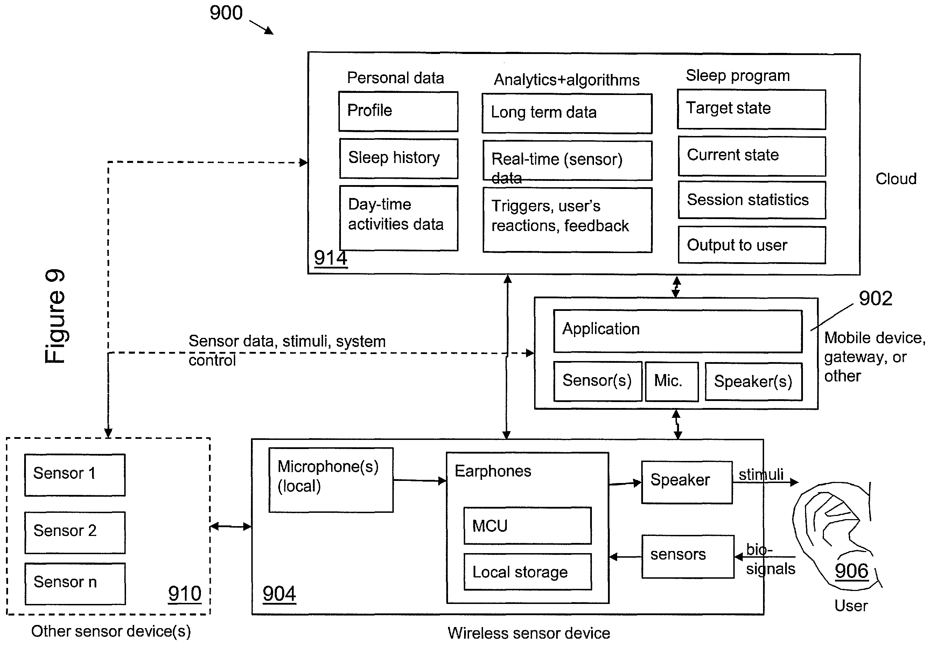

Figure 9 shows an example system according to the present disclosure similar to that shown in Figure 8. Figure 9 shows an example system 900 according to the present disclosure. The system comprises at least a control apparatus 902, and one or a pair of earphones 904 in communication with the control apparatus 902. The control apparatus 902 is configured to receive a sleep profile of a user 906, the sleep profile comprising recorded sleep phases of a sleep session of the user 906; receive a target sleep outcome for the user 906, and based on the sleep profile and the target sleep outcome, determine a sleep adjustment for provision to the user 906. The sleep adjustment comprises (in this example, audio) stimuli configured to attempt to adjust the user's sleep during one or more of the sleep session and a subsequent sleep session in an attempt to, at least in part, achieve the target sleep outcome. The control apparatus 902 is also configured to provide the determined sleep adjustment to the one or a pair of earphones 904. The one or a pair of earphones 904 is configured to receive the determined sleep adjustment comprising the stimuli from the control apparatus 902, and provide the audio stimuli to the user 906 during the subsequent sleep session.

The one or a pair of earphones 904 in this example is comprised in a wireless sensor device 904 comprising the earphones, which themselves comprise a microcontroller unit (MCU) and a local storage unit/memory. The one or a pair of earphones 904 also in this example comprises a local microphone which is configured to detect sounds from the user and/or the ambient surroundings, a speaker configured to provide sounds (audio stimuli) to the user, and one or more sensors/biosensors to detect biosignals from the user, for example to determine the user's sleep habits/phases. The sleep biosignal may comprise one or more of: a position of the user's head; a position of the user's body; motion of the user's head; motion of the user's body; motion of the user's eyes; the heart rate of the user; R-R intervals of the user's heartbeats; the breathing rate of the user; rapid eye movement detection (e.g. using a camera); and the temperature of the user during the sleep session.

A further sensor device 910 may be in communication with the wireless sensor device 904, and be configured to provide one or more other sleep related functions, such as determining a biosignal from the user indicative of sleep. The device 910 may be worn by the user (e.g. a smartwatch, body strap such as a chest strap, or embedded into a garment to be worn by the user), may be proximal to the sleeping user (e.g. in, on or under a pillow, mattress, blanket, or headboard/bedframe), or may be remote from the user (e.g. a motion detector, IR camera, or ambient thermometer). Another possible function of the device 910 is to provide one or more stimuli to the user during sleep to help achieve the user's target sleep outcome (e.g. ambient lighting to induce a particular sleep phase). A wearable device 910 may also be used to detect one or more biosignals during the time the user is awake, for example by measuring when the user is walking or running (e.g. a pedometer) or undergoing physical activity (e.g. by measuring changes in sweat produced and heart rate). Such daytime/awake time biosignals may form part of the user's sleep profile, for example, the sleep profile may indicate that the user generally falls into a deeper sleep more quickly if they have done cardio/physical exercise earlier that day.

The cloud/analysis module 914 may determine the user's sleep phases during a sleep session from data recorded by one or more sensors e.g. with the audio output device 904, and/or may determine and/or provide audio stimuli (and any other additional stimuli) to provide to the user by the earphones 904 during a sleep session to help achieve the target sleep outcome. The cloud/analysis module 914 in this example comprises a personal data file for the user, with the user's profile (e.g. name, age, sex, weight, height, ethnicity, profession, known medical issues, other demographics) the user's sleep history (e.g. recorded from one or more previous sleep sessions), and data on the user's daytime activities (e.g. time/duration spend sitting/resting, playing sports, walking, commuting, working etc).

The cloud/analysis module 914 in this example also comprises analytics and algorithms for recording and analysing user data, such as long-term data of the user (e.g. any changes in sleep habits detected since using the system 900), real-time sensor data (e.g. detected using the biosignal sensors 904 and/or from other remote sensors 910), and triggers, user reactions and feedback (e.g. current user reactions to provided audio stimuli, and detected biosignals associated with particular detected sleep habits, such as waking following a change in posture or snoring following the user moving into a deeper phase of sleep).

The cloud/analysis module 914 in this example also comprises a sleep program which uses the target state/target sleep outcome, and the current sleep state of the user, as well as session statistics (e.g. biosignals detected during the current sleep session) to determine an output to provide to the user to attempt to achieve the target sleep outcome. For example, the user may provide a target of wishing to sleep for longer. The current state may be that the user is in REM sleep at 6am. The session statistics may indicate that the user has had insufficient deep sleep in the current sleep session to feel that they have had enough sleep upon waking. The output provided to the user may then be determined to be the provision of audio stimuli (e.g. sounds from nature) to induce a deeper sleep in the user to ensure they do not wake from their current REM state but instead move into Stage 1 (or deeper) sleep, such that when they wake up, later than 6am, they have slept for longer and feel refreshed.

In some examples, the apparatus may be configured to determine whether the target sleep outcome has been achieved (and to what extent). For example, if the target sleep outcome is to feel more refreshed then one or more biosignals indicative of a user's wellbeing (e.g. heart rate, sweat production, blood pressure) may be monitored to check if the user is in a refreshed state following the sleep session. As another example, if the user's target sleep outcome is to improve their memory, and the apparatus attempted to achieve this goal by attempting to prolong the length of time spent in Stage 2 sleep, for example, then the user's sleep phases may be monitored using one or more biosensors to check if the user really did achieve a longer time in Stage 2 sleep. The user may, in some examples, be presented with a cognitive test, for example a memory test, on their smartphone or tablet computer following the sleep session, to check if the user's memory has improved from test results recorded from before the user underwent the sleep session in which stimuli were provided.

If the user's biosignals and/or test results indicate that significant progress has been achieved, then the user may continue to be provided with stimuli during sleep to progress further if appropriate (and the user's sleep profile may be updated to reflect the latest results). If the user's biosignals and/or test results indicate that progress has not been achieved sufficiently (e.g. less than a 20% increase in correct test answers, less than a 5% decrease in blood pressure at a particular time of day compared with the measurement taken before the sleep session(s)), then the user may, for example, be provided with different stimuli during sleep to attempt to make progress in achieving the target sleep outcome (and the user's sleep profile may be updated to reflect the latest results). In some examples, continuation of use of the apparatus for a particular target sleep outcome may depend on the user passing one or more post-sleep "tests" (e.g. quiz/test results, and/or particular biosignals being within a predefined range or improved by a predetermined factor).

In some examples, information on external factors may be received by the apparatus during the user's sleep session and taken as input to adjust the target sleep outcome and/or stimuli provided to the user during that sleep session. For example, if the weather forecast indicates that it is likely to rain in the morning after 9am, the user may receive stimuli to ensure they are awake at 8am to e.g. travel to work avoiding the rain. The accuracy of predicted weather conditions at a particular time may increase closer to the particular time, so the morning weather may be more accurately predicted closer to the morning (during the time the user is asleep) than in the previous evening or earlier. As another example, real-time (or near-real-time) traffic updates (such as indications of congested or blocked roads, or cancelled trains/flights) may be received prior to or during the user's sleep session and accounted for in determining the stimuli to be provided to the user. For example, if the user's target sleep outcome is to attend a meeting in the next town at 12 noon, and during the night the train line which the user would be planning to use (e.g. as determined from the user's calendar/emails) or which the user would be likely to use (e.g. determined from a train line map) is closed, the user may be provided with stimuli to cause them to, for example, wake up earlier than planned to catch an alternative train or consider driving instead.

In some examples, the apparatus may be configured to determine if, in examples where the user is wearing a wearable device such as an earphone, pair of earphones, or other wearable biosensor, if the item is correctly positioned with respect to the user. If, for example, the user's earphone(s) fall out of the user's ears, then the provision of audio stimuli via the displaced earphone(s) may be e.g. stopped, changed (for example, stereo sound from two earphones may be changed to mono sound provided by one earphone remaining in the user's ear while the displayed earphone is not used), or a power-save mode may be entered.

In some examples, the apparatus (e.g. apparatus 802, 902) may be configured to determine whether the duration of provision of the sleep adjustment exceeds a predetermined sleep adjustment threshold, and if it does, prevent the provision of further sleep adjustment/audio stimuli until the expiry of a sleep adjustment cooling off period. The predetermined sleep adjustment threshold may be a predetermined number of consecutive sleep sessions. For example, to prevent a user becoming dependent on using the apparatus such that healthy sleep without use of the apparatus is not possible, the apparatus may determine that, for example, following seven consecutive nights use to be provided with audio stimuli, the user may not use the device again for a 72-hour period.

Or, if the user wishes to achieve a target sleep outcome for a particular date (e.g. the date of an important meeting) the sleep training/provision of audio stimuli will not begin earlier than three sleep sessions prior to the meeting and may not be used again following the meeting for 48 hours.

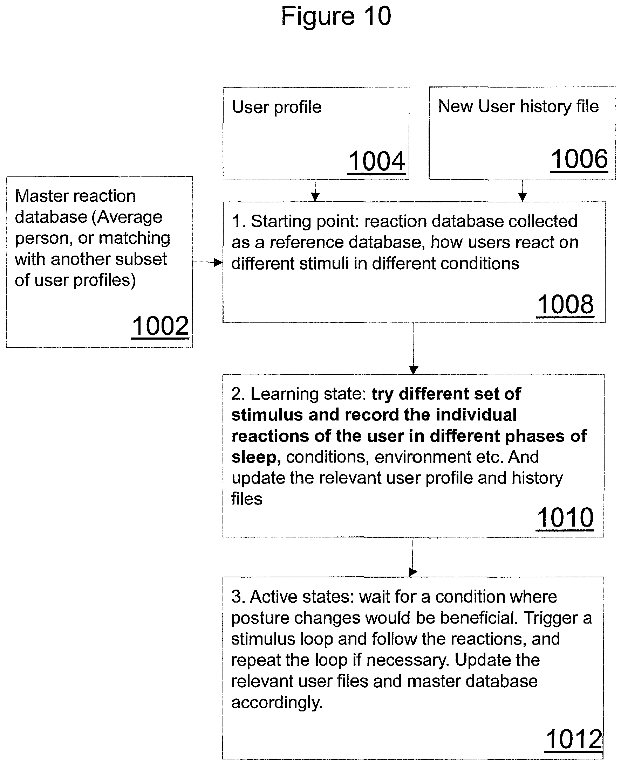

Figure 10 shows an example method for determining sleep adjustments for a user according to the present disclosure. Initially, the user profile 1004, and the history of the new user 1006 are taken as input. Also, at the start, a master reaction database 1002 is used, which is a database of behaviour and reactions in relation to sleep of the "average person", for example a person of a similar demographic group to the new user. As a starting point/first step 1008, a reaction database collected as a reference database, which indicates/logs how a user reacts to different stimuli in different conditions. Next is a learning stage 1010, during which different sets of stimuli are tired on the user, and the individual reactions of the user are recorded, in different phases of sleep, conditions, environment etc. The user profile and history files are then updated with this experiment/test information. Finally, in an active state 1012, the method comprises waiting for a condition of the user (e.g. a sleep disorder such as snoring, or a detected unfavourable posture for sleeping) where posture changes would be beneficial. A stimulus loop is then triggered, and the reaction of the user can be followed. This process may be repeated if necessary. The relevant user files and master database are then updated accordingly following provision of the stimuli. In this way the user profile may be tuned to the particular behaviour and requirements of the user to better provide effective audio stimuli to induce sleep adjustments for the user.

While the above discussion uses examples relating to a night's sleep, the examples may also apply equally well for a daytime sleep session (for example, for a user who works night shifts), to users who are changing time zones (for example, business travellers flying between time zones), and to user's for whom the "sleep session" may not be the predominant sleep session for a 24 our period, but may be a "power nap" session of a short period of sleep time (for example, a nurse who is working a night shift, wants to have a two/three-hour "power nap" sleep session, before awaking to spend time with the family in the morning before an afternoon at work). In some examples, the apparatus may be configured so that a resting period of a user may be considered to comprise more than one sleep session. For example, if a user wakes up twice during a single night's sleep, then it may be determined that the period of rest comprises three distinct sleep sessions.

In some examples, a sleep adjustment may be configured to attempt to adjust a user's sleep during a subsequent sleep session (e.g. following a period of sleep monitoring, or following user provision of a target sleep outcome earlier that day). The "subsequent" nature may be, therefore, following one or more previous sleep sessions, and/or following input prior to the sleep session in which the stimuli are to be provided to the user.

In some examples, a sleep adjustment may be configured to attempt to adjust a user's sleep at a subsequent time during a current sleep session. That is, the apparatus may be configured to dynamically adjust a users' sleep "on the fly", during the "current" sleep session, based on the sleep profile (and optionally biosignals) recorded for the user during a current sleep session. As an example, if a user moves into a sleeping position which is an uncomfortable sleep position (e.g. with the neck bent), this may be detected by e.g. a head-worn gyroscope sensor, and the apparatus may determine and provide stimuli in response to the detection of movement/posture, to induce the user to change to a more comfortable sleeping posture (e.g. neck straight).

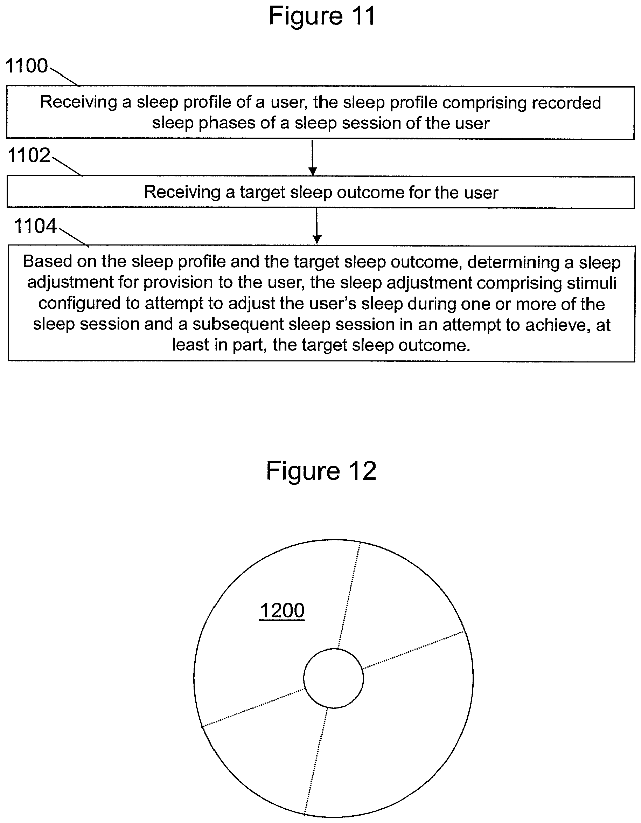

Figure 11 shows an example method according to the present disclosure. The computer-implemented method comprises, receiving a sleep profile of a user, the sleep profile comprising recorded sleep phases of a sleep session of the user; receiving a target sleep outcome for the user, and based on the sleep profile and the target sleep outcome, determining a sleep adjustment for provision to the user, the sleep adjustment comprising stimuli configured to attempt to adjust the user's sleep during one or more of the sleep session and a subsequent sleep session in an attempt to achieve, at least in part, the target sleep outcome.

Figure 12 shows an example computer-readable medium comprising a computer program configured to perform, control or enable the method of Figure 10, 11 or any method described herein. The computer program may comprise computer code configured to perform the method(s), such as the method of receiving a sleep profile of a user, the sleep profile comprising recorded sleep phases of a sleep session of the user; receiving a target sleep outcome for the user, and based on the sleep profile and the target sleep outcome, determining a sleep adjustment for provision to the user, the sleep adjustment comprising stimuli configured to attempt to adjust the user's sleep during one or more of the sleep session and a subsequent sleep session in an attempt to achieve, at least in part, the target sleep outcome.

In this example, the computer/processor readable medium 1200 is a disc such as a digital versatile disc (DVD) or a compact disc (CD). In other examples, the computer/processor readable medium 1200 may be any medium that has been programmed in such a way as to carry out an inventive function. The computer/processor readable medium 1200 may be a removable memory device such as a memory stick or memory card (SD, mini SD, micro SD or nano SD card).

It will be appreciated to the skilled reader that any mentioned apparatus/device and/or other features of particular mentioned apparatus/device may be provided by apparatus arranged such that they become configured to carry out the desired operations only when enabled, e.g. switched on, or the like. In such cases, they may not necessarily have the appropriate software loaded into the active memory in the non-enabled (e.g. switched off state) and only load the appropriate software in the enabled (e.g. on state). The apparatus may comprise hardware circuitry and/or firmware. The apparatus may comprise software loaded onto memory. Such software/computer programs may be recorded on the same memory/processor/functional units and/or on one or more memories/processors/functional units.

In some examples, a particular mentioned apparatus/device may be pre-programmed with the appropriate software to carry out desired operations, and wherein the appropriate software can be enabled for use by a user downloading a "key", for example, to unlock/enable the software and its associated functionality. Advantages associated with such examples can include a reduced requirement to download data when further functionality is required for a device, and this can be useful in examples where a device is perceived to have sufficient capacity to store such pre-programmed software for functionality that may not be enabled by a user.

It will be appreciated that any mentioned apparatus/circuitry/elements/processor may have other functions in addition to the mentioned functions, and that these functions may be performed by the same apparatus/circuitry/elements/processor. One or more disclosed aspects may encompass the electronic distribution of associated computer programs and computer programs (which may be source/transport encoded) recorded on an appropriate carrier (e.g. memory, signal).

It will be appreciated that any "computer" described herein can comprise a collection of one or more individual processors/processing elements that may or may not be located on the same circuit board, or the same region/position of a circuit board or even the same device. In some examples one or more of any mentioned processors may be distributed over a plurality of devices. The same or different processor/processing elements may perform one or more functions described herein.

It will be appreciated that the term "signalling" may refer to one or more signals transmitted as a series of transmitted and/or received signals. The series of signals may comprise one, two, three, four or even more individual signal components or distinct signals to make up said signalling. Some or all of these individual signals may be transmitted/received simultaneously, in sequence, and/or such that they temporally overlap one another.

With reference to any discussion of any mentioned computer and/or processor and memory (e.g. including ROM, CD-ROM etc.), these may comprise a computer processor, Application Specific Integrated Circuit (ASIC), field-programmable gate array (FPGA), and/or other hardware components that have been programmed in such a way to carry out the inventive function.

The applicant hereby discloses in isolation each individual feature described herein and any combination of two or more such features, to the extent that such features or combinations are capable of being carried out based on the present specification as a whole, in the light of the common general knowledge of a person skilled in the art, irrespective of whether such features or combinations of features solve any problems disclosed herein, and without limitation to the scope of the claims. The applicant indicates that the disclosed examples may consist of any such individual feature or combination of features. In view of the foregoing description it will be evident to a person skilled in the art that various modifications may be made within the scope of the disclosure.

While there have been shown and described and pointed out fundamental novel features as applied to different examples thereof, it will be understood that various omissions and substitutions and changes in the form and details of the devices and methods described may be made by those skilled in the art without departing from the spirit of the invention. For example, it is expressly intended that all combinations of those elements and/or method steps which perform substantially the same function in substantially the same way to achieve the same results are within the scope of the invention. Moreover, it should be recognized that structures and/or elements and/or method steps shown and/or described in connection with any disclosed form or example may be incorporated in any other disclosed or described or suggested form or example as a general matter of design choice. Furthermore, in the claims means-plus-function clauses are intended to cover the structures described herein as performing the recited function and not only structural equivalents, but also equivalent structures. Thus, although a nail and a screw may not be structural equivalents in that a nail employs a cylindrical surface to secure wooden parts together, whereas a screw employs a helical surface, in the environment of fastening wooden parts, a nail and a screw may be equivalent structures.

## Claims

1. An apparatus comprising:
at least one processor; and
at least one memory including computer program code,
the at least one memory and the computer program code configured to, with the at least one processor, cause the apparatus to perform at least the following:
receive a sleep profile of a user, the sleep profile comprising recorded sleep phases of a sleep session of the user;
receive a target sleep outcome for the user, and
based on the sleep profile and the target sleep outcome, determine a sleep adjustment for provision to the user, the sleep adjustment comprising stimuli configured to attempt to adjust the user's sleep during one or more of the sleep session and a subsequent sleep session in an attempt to achieve, at least in part, the target sleep outcome.

2. The apparatus of claim 1, wherein the memory and computer program code are configured to, with the processor, cause the apparatus to determine the sleep adjustment for provision to the user by using a sleep biosignal recorded during a sleep session of the user.

3. The apparatus of claim 2, wherein the sleep biosignal comprises one or more of:
a position of the user's head;
a position of the user's body;
motion of the user's head;
motion of the user's body;
motion of the user's eyes;
the heart rate of the user;
R-R intervals of the user's heartbeats;
the breathing rate of the user; and
the temperature of the user
during the sleep session.

4. The apparatus of any preceding claim, wherein the target sleep outcome is one or more of:
determined by the apparatus based on the received sleep profile;
input by the user; and
input by a secondary user other than the user.

5. The apparatus of any preceding claim, wherein the target sleep outcome is one or more of:
sleeping for a different total sleep time, in a subsequent sleep session, than the sleep profile total sleep time;
sleeping for the same total sleep time, but for a different portion of the day, in a subsequent sleep session, compared with the portion of the day spent sleeping of the sleep profile;
changing the length of time of one or more sleep phases;
changing the length of time of one or more sleep phases in a subsequent sleep session compared with the corresponding sleep phases of the sleep profile; and
reducing the duration of one or more detected adverse sleep conditions.

6. The apparatus of any preceding claim, wherein the memory and computer program code are configured to, with the processor, cause the apparatus to
receive awake profile data for the user, the awake profile data comprising a record of activities during a period of being awake; and
determine the sleep adjustment further based on the awake profile data for the user.

7. The apparatus of any preceding claim, wherein the sleep adjustment comprises stimuli configured to attempt to adjust the user's sleep during one or more of the sleep session and the subsequent sleep session by attempting to induce an adjustment in sleeping posture of the user.

8. The apparatus of any preceding claim, wherein the stimuli are configured to be provided to the user by one or a pair of earphones to be worn by the user during one or more of the sleep session and the subsequent sleep session.

9. The apparatus of any preceding claim, wherein the apparatus is one of: an earphone, a module for an earphone, a server remote from and in communication with an earphone; the earphone to be worn by the user during one or more of the sleep session and the subsequent sleep session; or a pillow.

10. The apparatus of any preceding claim, wherein the sleep adjustment comprises one or more of: audio stimuli, vibratory stimuli, temperature stimuli, and pressure stimuli.

11. The apparatus of any preceding claim, wherein the memory and computer program code are configured to, with the processor, cause the apparatus to determine an awake instruction for provision to the user, the awake instruction indicating an activity for the user to perform to support the attempt to adjust the user's sleep during one or more of the sleep session and the subsequent sleep session in the attempt to achieve, at least in part, the target sleep outcome.

12. The apparatus of any preceding claim, wherein the memory and computer program code are configured to, with the processor, cause the apparatus to determine the identity of the particular user by determining one or more biosignals indicative of the particular user, and determine a sleep adjustment for the particular user.

13. A system comprising:
a control apparatus; and
one or a pair of earphones in communication with the control apparatus;
the control apparatus configured to:
receive a sleep profile of a user, the sleep profile comprising recorded sleep phases of a sleep session of the user;
receive a target sleep outcome for the user,
based on the sleep profile and the target sleep outcome determine a sleep adjustment for provision to the user, the sleep adjustment comprising stimuli configured to attempt to adjust the user's sleep during one or more of the sleep session and a subsequent sleep session in an attempt to achieve, at least in part, the target sleep outcome; and
provide the determined sleep adjustment to the one or a pair of earphones;
the one or a pair of earphones configured to:
receive the determined sleep adjustment comprising the stimuli from the control apparatus, and
provide the stimuli to the user during the one or more of the sleep session and the subsequent sleep session.

14. A computer-implemented method comprising:
receiving a sleep profile of a user, the sleep profile comprising recorded sleep phases of a sleep session of the user;
receiving a target sleep outcome for the user, and
based on the sleep profile and the target sleep outcome, determining a sleep adjustment for provision to the user, the sleep adjustment comprising stimuli configured to attempt to adjust the user's sleep during one or more of the sleep session and a subsequent sleep session in an attempt to achieve, at least in part, the target sleep outcome.

15. A computer readable medium comprising computer program code stored thereon, the computer readable medium and computer program code being configured to, when run on at least one processor, perform:
receiving a sleep profile of a user, the sleep profile comprising recorded sleep phases of a sleep session of the user;
receiving a target sleep outcome for the user, and
based on the sleep profile and the target sleep outcome, determining a sleep adjustment for provision to the user, the sleep adjustment comprising stimuli configured to attempt to adjust the user's sleep during one or more of the sleep session and a subsequent sleep session in an attempt to achieve, at least in part, the target sleep outcome.
